# EUROPEAN PATENT SPECIFICATION

(11) **EP 2 496 200 B1**
(45) Date of publication and mention of the grant of the patent: **11.12.2013**
(21) Application number: 10788160.9
(22) Date of filing: 24.09.2010
(51) Int. Cl.: A61F 13/62

(54) **HIGH-STRENGTH LOOP PIECE FORMED TO BE USED ON THE STRAPS OF DIAPERS FOR BABIES AND ADULTS**
HOCHFESTES SCHLEIFENTEIL ZUR VERWENDUNG AUF DEN FIXIERBÄNDERN VON WINDELN FÜR KLEINKINDER UND ERWACHSENE
ÉLÉMENT À BOUCLES GRANDE RÉSISTANCE FORMÉ POUR ÊTRE UTILISÉ SUR DES BANDES DE COUCHES POUR BÉBÉS ET ADULTES

(30) Priority: 06.11.2009 TR 200908430
(43) Date of publication of application: 12.09.2012
(73) Proprietor: Korozo Ambalaj Sanayi Ve Ticaret Anonim Sirketi, 34522 Istanbul (TR)
(72) Inventor: YAKAR, Cavit, 34522 Istanbul (TR)
(74) Representative: Iskender, Ibrahim
(86) International application number: PCT/TR2010/000190
(87) International publication number: WO 2011/056157

(56) References cited:
- EP-A1- 1 997 942
- WO-A1-2007/114362
- TR-A2- 200 706 579

## Description

### Technical Field

The invention is related to a loop piece on the straps used for mechanical opening/closing structures enabling the link between the front and back parts of baby diapers and adult diapers.

The invention, as said loop piece, is especially related to use a looped nonwoven fabric obtained by forming separate loop on a loopless and nonwoven fabric and related to the method thereof.

### The Related Art

According to the present technique, there are different structures used to combine the front and back sides of baby diapers and adult diapers. These structures are divided into two as adhesive and mechanical.

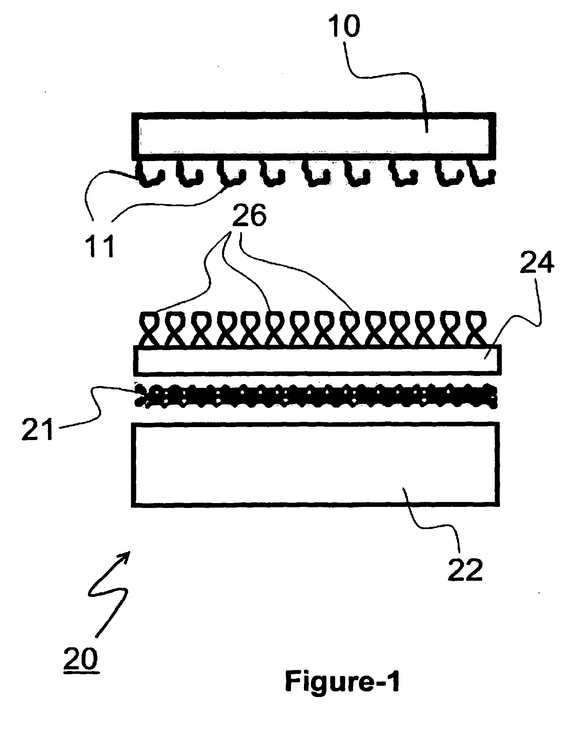

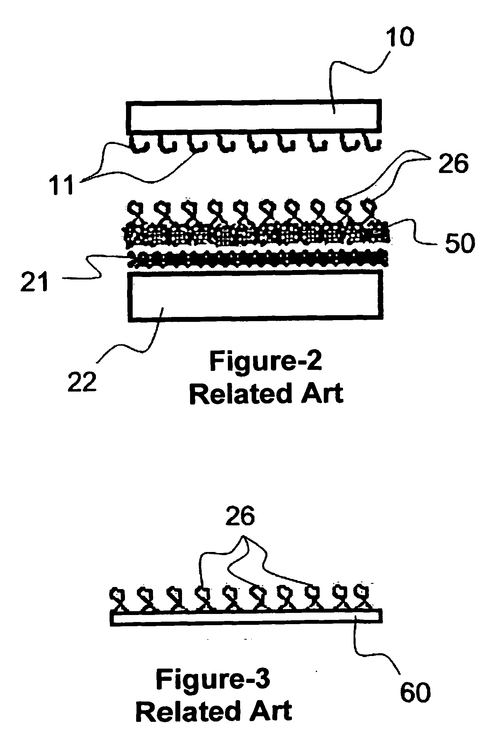

The closing structures defined as mechanical and used in the present technique are given in the Figure-2 and 3. The mechanical closing structures have a form made of plastic named hooked piece on both side tapes of the baby diaper. The said hooked piece enables fitting hooks and loops together when said piece is combined with the looped fabric on the front of the diaper.

The present loop piece in general is comprised of looped fabric and a thermoplastic layer. The loop fabric may be a fabric manufactured by knitting, weaving or other known techniques or may be nonwoven (Figure-2).

As an example for this, the American patent publication no. US 2005003143 is related to the method of establishing a layered composite structure comprised of a film made of standard synthetic material and looped knitted or nonwoven fabric for manufacturing especially the hooked and loop closing parts of diapers. The patent publication no. JP 2005253649 is related to the fine and perfectly elastic knitted fabric on the hooked and loop connection parts on diapers. Finally, the European patent publication no. EP 1350879 is related to the warp knitted textile fabric and the manufacturing method of this fabric. This fabric is related to the new warp knitted loop fabric used as a loop component in especially hooked and loop type, two-component fabric fastener.

An adhesive-containing interlayer as a result of combining said loop fabric and the printed or printless plastic layer through various methods such as coating, extrusion draining, glued lamination etc.

In these combination systems, in cases the printed or printless plastic layer is directly combined, the thermoplastic material is melted and then applied, therefore it is fluid.

In the glued combination according to another method, the glue is liquid. These materials penetrate into loops, since they are liquid and prevent some of the loops from being free. This reduces the adherence strength of the loops, which is an unwanted result. When the glue is used less in order to reduce its penetration, the adherence strength gets low and the layers can be easily separated under pressure.

The patent publication no. TR 2007 06579 is related to a loop piece developed to solve said problem. In this piece, the loops are formed on a thermoplastic film base (Figure-3). The thermoplastic film base functions as a barrier between the printed or printless plastic layer and the loops that prevents the glue from penetration into the loops. The problem with this method is the reducing mechanical strength during the process of loop making.

Due to all these unfavorable facts, the need for making a high-strength loop piece was born.

### Short Description of the Invention

The invention is made being inspired by the present situations and aims to solve the above-mentioned problems.

The primary objective of the invention is while forming a loop piece used on mechanical opening/closing structures enabling the combination of the front and back sides of the baby and adult diapers, to prevent the liquid thermoplastic piece or the adhesive material from flowing into the loops, some of the loops from being released and the adherence strength of the loops from reducing.

The invention aims to present a loop piece that is stronger than the present loop thermoplastic film in terms of breaking strength and tearing strength.

An object of the invention is to enable the diapers to enwrap the user in the best tightness and durability.

The structural and characteristic features of the invention and all its advantages will be understood more clearly with the figures given below and the detailed description written by referring to these figures and therefore, the evaluation should be done by taking these figures and the detailed description into consideration.

### Figures to Help Understanding the Invention

Figure - 1 represents the layers of the strap that has the loop piece that is the subject of the invention.
Figure - 2 represents the layers of the strap that has a self-looped fabric of the previous technique.
Figure - 3 presents the representative side view of the loop thermoplastic film of the previous technique.

The drawings do not have to be scaled and thus, unnecessary details might be disregarded in order to understand the present invention. Moreover, the elements that are at least substantially identical or having at least substantially same functions are shown with the same number.

**Description of the Reference Numbers**

| | | | |
|---|---|---|---|
| **10.** | Hooked piece | **50.** | Self-looped fabric |
| **11.** | Hook | | |
| | | **60.** | Thermoplastic film |
| **20.** | Loop piece | | |
| **21.** | Adhesive | | |
| **22.** | Plastic layer | | |
| **24.** | Loopless nonwoven surface or fabric | | |
| **26.** | Loop | | |

### Detailed Description of the Invention

In this detailed description, the preferred structures of the loop piece that is the subject of the invention are explained only for the purposes of understanding the subject better and without reservation.

The invention is related to a loop piece (20) used on straps having at least one hooked piece (10) to connect the front and back sides of especially baby diapers and adult diapers and formed as the counterpart for said hooked piece (10) to fasten. The novelty of the invention is that the loop piece (20) has at least one loopless nonwoven surface or fabric (24) and at least one loop (26) separately formed on it.

### The preferred structure of the invention:

The nonwoven surfaces or fabrics (24) may be made preferably of, but not limited to thermoplastics such as polypropylene, polyester, viscose etc. or their specific combinations.

The loop (26) on the nonwoven or fabric (24) can be made in various thicknesses in line with the requirements of the application. For forming the loops (26), various thread types can be used made of polyamide, polyester etc. or their specific combinations.

In the preferred application, the plastic layer (22) fixed under the loop piece (20) and formed as printed or printless is also preferably made of thermoplastic materials.

The process of forming a loop (26) on said nonwoven surface or fabric (24) is realized by knitting on the knitting machine or in similar ways. The preferred technique is securing the nonwoven surface (24) through the sewing - knitting technique and sewing a thread on the nonwoven surface (24) in a chain. However, other production techniques can also be used. The primary aspect here is that the loops (26) do not form fabric on its own, but are on a nonwoven surface or fabric (24) base, rather than on a thermoplastic film (60).

Figure-1 presents the side view of the whole loop nonwoven fabric (24) and the loop piece (20). The looped nonwoven fabric (24) is then combined with a plastic layer (22). This second layer (22) may be printed or printless. The phase of combining the looped nonwoven fabric (24) and the plastic layer (22) is carried out by laminating by adhesives like glue etc. (21) or draining / coating by the thermoplastic material extrusion method.

The protection scope of this application is identified in the claims part and it cannot be restricted to what explained above for exemplification.

## Claims

1. A loop piece (20) used on straps having at least one hooked piece (10) to connect the front and back sides of especially baby diapers and adult diapers and formed as the counterpart for said hooked piece (10) to fasten and **characterized in that** it comprises of at least one loopless nonwoven surface or fabric (24) and at least one loop (26) individually formed on said loopless nonwoven or fabric (24) in a manner of that said loop (26) does not form fabric on its own, but it is on the nonwoven or fabric surface (24) base.

2. The loop piece (20) according to the Claim 1, wherein the nonwoven surface or fabric (24) is **characterized by** being manufactured of thermoplastic material.

3. The loop piece (20) according to the Claim 1 or 2, wherein, said nonwoven surface or fabric (24) is **characterized by** being manufactured of a material selected from the group of polypropylene, polyester, viscose and their combinations.

4. The loop piece (20) according to any of the previous claims, wherein said loop (26) is **characterized by** being manufactured of a material selected from the group of polyamide, polyester and their combinations.

## Patentansprüche

1. Schleifenstück (20) zum Gebrauch an Bändern mit einem Hakenstück (10) zum Verbinden der Vorder- und Rückseite besonders von Babywindeln und Erwachsenenwindeln, das als das Gegenstück für das Hakenstück (10) zum Befestigen ausgebildet ist und **dadurch gekennzeichnet ist, dass** es zumindest eine schleifenlose Vliesoberfläche oder einen Stoff (24) und zumindest eine Schleife (26) umfasst, die derart individuell auf dem schleifenlosen Vlies oder Stoff (24) ausgebildet ist, dass die Schleife (26) selbst keinen Stoff ausbildet, sondern sich auf der Basis der Vlies- oder Stoffoberfläche (24) befindet.

2. Schleifenstück (20) nach Anspruch 1, wobei die Vliesoberfläche oder der Stoff (24) **dadurch gekennzeichnet ist, dass** sie/er aus Thermoplastmaterial hergestellt ist.

3. Schleifenstück (20) nach einem der Ansprüche 1 oder 2, die Vliesoberfläche oder der Stoff (24) **dadurch gekennzeichnet ist, dass** sie/er aus einem Material hergestellt ist, das aus der Gruppe von Polypropylen, Polyester, Viskose und ihren Kombinationen ausgewählt ist.

4. Schleifenstück (20) nach einem der vorhergehenden Ansprüche, wobei die Schleife (26) **dadurch gekennzeichnet ist, dass** sie aus einem Material hergestellt ist, das aus der Gruppe von Polyamid, Polyester und ihren Kombinationen ausgewählt ist.

## Revendications

1. Pièce de boucle (20) utilisée sur des attaches possédant au moins une pièce de crochet (10) destinée à relier les côtés avant et arrière, notamment sur des couches pour bébé et des couches pour adulte, et formée comme équivalent de ladite pièce de crochet (10) pour fixer, **caractérisée en ce qu'**elle comprend au moins une surface ou un textile non-tissé(e) sans boucle (24) et au moins une boucle (26) formée individuellement sur ladite surface ou ledit textile non-tissé(e) sans boucle (24), de manière à ce que ladite boucle (26) ne forme pas un textile en elle-même, mais se trouve sur la base de la surface de textile ou de non-tissé (24).

2. Pièce de boucle (20) selon la revendication 1, dans laquelle la surface ou le textile non-tissé(e) (24) est caractérisé(e) en ce qu'il/elle est fabriqué(e) à partir d'une matière thermoplastique.

3. Pièce de boucle (20) selon la revendication 1 ou 2, dans laquelle la surface ou le textile non-tissé(e) (24) est **caractérisé en ce qu'**il/elle est fabriqué(e) à partie d'une matière sélectionné parmi le groupe comprenant le polypropylène, le polyester, la viscose et leurs combinaisons.

4. Pièce de boucle (20) selon l'une quelconque des revendications précédentes, dans laquelle ladite boucle (26) est **caractérisée en ce qu'**elle est fabriquée à partir d'une matière sélectionnée parmi le groupe comprenant le polyamide, le polyester et leurs combinaisons.
